# EUROPEAN PATENT SPECIFICATION

(11) **EP 2 512 402 B1**
(45) Date of publication and mention of the grant of the patent: **25.03.2015**
(21) Application number: 10790967.3
(22) Date of filing: 14.12.2010
(51) Int. Cl.: A61C 17/00, A61K 8/04, A61K 8/22, A61K 8/29, A61Q 11/00, A61Q 11/02

(54) **DEBRIDEMENT PASTE**
DEBRIDEMENTPASTE
PÂTE DE DÉBRIDEMENT

(30) Priority: 15.12.2009 EP 09179258
(43) Date of publication of application: 24.10.2012
(73) Proprietor: Corticalis AS, 1450 Nesoddtangen (NO)
(72) Inventor: LYNGSTADAAS, S., Petter, N-1450 Nesoddtangen (NO); HAUGEN, Håvard, J., N-0376 Oslo (NO)
(74) Representative: Valea AB
(86) International application number: PCT/EP2010/069638
(87) International publication number: WO 2011/073194

(56) References cited:
- EP-A1- 1 192 933
- WO-A1-2009/083281
- DE-A1- 10 034 916
- US-A- 5 045 318
- US-A- 5 885 553
- US-A1- 2002 104 269
- US-A1- 2008 118 447

## Description

### Technical field

The present invention relates to a new and inventive composition for implant cleaning and debridement of hard surfaces in the oral cavity, which comprises optimally activated nanoparticles of TiO₂, and H₂O₂, being antibacterial, without causing microbial resistance, and anti-inflammatory, and wherein said composition further comprises solid microparticles for improved mechanical debridement and/or cleaning of rough surfaces.

The present invention further relates to the use of a composition according to the present invention for cleaning an implant and/or debriding a hard surface in the oral cavity.

In a presently preferred embodiment, the composition according to the present invention is used together with an implant cleaning and/or debridement tool, e.g. a brush, burr or a curette.

### Background

With the increased use of osseointegrated implants and with many implants functioning for long periods of time, various complications have been reported. Progressive loss of peri-implant bone is one of the major concerns during the function period of implants. The recognition and treatment of peri-implant bone loss around functioning implants is a major challenge for the clinician.

Diagnostic techniques, such as probing pocket depth, radiographic tools, and microbial sampling have been modified from the periodontal area and used during the maintenance phase of the dental implant. The long-term goals in the treatment of peri-implant disease are to arrest the progression of the disease and to achieve a maintainable site for the patient's implant.

Recent reports indicate that peri-implant bony defects can be treated with either nonsurgical or surgical techniques. Bone regeneration is possible in selected peri-implant bony defects when appropriate surgical techniques are used, implant surface preparation is achieved, and the cause is eradicated.

Debridement is the medical removal of a patient's dead, damaged, or infected tissue to improve the healing potential of the remaining healthy tissue. Removal may be surgical, mechanical, chemical, autolytic (self-digestion), and by maggot therapy, where certain species of live maggots selectively eat only necrotic tissue.

In oral hygiene and dentistry, debridement also refers to the removal of plaque and calculus that have accumulated on the teeth, or any other hard tissue surface in the oral cavity. Debridement in this case may be performed using ultrasonic instruments, which fracture the calculus, thereby facilitating its removal, as well as hand tools, including periodontal scaler and curettes, brushes or through the use of chemicals, such as hydrogen peroxide.

Debridement is an important part of the treatment process for healing of peri-implant bone loss.

Many medical implants, such as e.g. dental implants, orthopedic implants and vascular stents, are metallic, i.e. they are made of a metal material. Examples of metal materials commonly utilized for constructing metallic medical implants are steel, titanium, zirconium, tantalum, niobium, hafnium and alloys thereof. In particular, titanium and titanium alloys have proved to be suitable to utilize for constructing medical implants. This is due to the fact that titanium is biocompatible, it has excellent corrosion resistance in body fluids, it resists adherence of bacteria, and it is light and strong.

Traditionally, the dentists and surgeons utilize cleaning tools that are relatively hard, i.e. they have a high hardness degree, in order to provide a thorough cleaning of the metallic medical implant during e.g. surgery, implantation or other treatments. Such hard cleaning tools may, for example, be made of stainless steel, hard metal alloys or hard polymers. However, such hard cleaning tools are not suitable to utilize for cleaning all metallic implant materials. For example, they are not suitable to utilize for cleaning medical implants of softer metals or metal alloys, such as e.g. titanium, a titanium alloy, zirconium or a zirconium alloy. This is due to the fact that such medical implants have a delicate surface that may be damaged when contacted by hard cleaning tools. Thus, when hard cleaning tools are utilized for cleaning a medical implant of, for example, titanium, a titanium alloy, zirconium or a zirconium alloy there is a great risk that the surface of the medical implant is damaged by the cleaning process. Then the surface structure of the medical implant is negatively affected. In addition, any produced scratches in the medical implant surface may constitute sites in which bacteria may adhere, which may result in reinfections in the tissue surrounding the medical implant, e.g. the gingiva.

Furthermore, the above mentioned hard cleaning tools may contaminate a delicate surface of a medical implant when utilized for cleaning the medical implant surface, i.e. they may leave contaminating material residues on the medical implant surface. These material residues often trigger a foreign body response and are generally not well accepted by the human body.

In order to avoid the above mentioned damaging risk, a cleaning tool in the form of a brush comprising soft bristles may be utilized instead of the above mentioned hard cleaning tools for cleaning metallic medical implants having delicate surfaces. One example of such a brush for cleaning a dental implant is disclosed in US 6,345,406, another example is given in WO 2009/083281, which discloses the implant cleaning/debridement tool TiBrush™.

The success rate for implanted titanium devices is affected by several factors. Among these, the surface properties of the implants seem to be a very important factor for a good clinical outcome. The body mainly interacts with the surface of the implant. The chemical-composition, topography, roughness and surface-energy have all been suggested to play important roles in implant bone interaction. These properties are highly interrelated, and it is not always easy to separate their effects.

Most implants used today have surfaces modified by machining, blasting, acid etching, or by combinations of these procedures. Today, machined and grit blasted surfaces are the most commonly used for surface treatment of titanium implants. Machined surfaces are considered relatively smooth with reported surface values in the range of Ra: 0.15-0.53 µm. The roughness of grit blasted and/or etched titanium surfaces are mostly in the µm range, with typical Ra range between 0.5 µm - 10 µm.

Consequently, the usefulness of a brush to clean and/or debride an implant surface effectively will correlate to the thickness of its bristles, which are in WO 2009/083281 typically given to have a length of e.g. 0.1-50 mm or 0.1-10 mm and a diameter of 0.05-1.0 mm or 0.05-0.5 mm. I.e., the bristles are in themselves too thick to be able to access the part or the parts of the surface that is hidden inside the roughness of the implants.

What is more, a brush is of course only able to perform the mechanical cleaning and/or debridement of a surface, without being able to provide any secondary chemical and/or biological cleaning/decontamination effect, thus e.g. leaving the surface open for immediate repopulation of microbes, or even leaving traces of the prior microbial populations, e.g. on the inaccessible areas of rough surfaces.

TiO₂, titanium (IV) oxide or titania is the naturally formed oxide of titanium and a very well-known and well-researched material due to the stability of its chemical structure, its biocompatibility, and physical, optical and electrical properties. Titanium dioxide occurs in nature as the well-known naturally occurring minerals rutile, anatase and brookite. It has previously been disclosed that TiO₂, when activated by UV-light, forms the free radical TiO-OH⁻.

UV-activated TiO₂ is a promising technique for decontamination, purification, and deodorization of air and wastewater (Yu et al. 2001) and has also been applied to inactive bacteria, viruses and cancer cells (Sunada et al 2003). Also, TiO-OH⁻ is disclosed to have an anti-fouling and antibacterial effect (Byrne et al. 1998, Maness et al. 1999, Yu et al. 2001).

It has also been previously shown that ceramic TiO₂ may be activated to form the free radical TiO-OH⁻ in the presence of H₂O₂. Silva et al. (Silva et al. 2007) used nanoparticles of TiO₂ for treatment of olive mill wastewater by activating the TiO₂ particles by using a combination of very low amounts of H₂O₂ (up to 0.5 mM) and UV-light.

In WO 89/06548, it is disclosed that the reaction product of H₂O₂ and metallic titanium, a gel, may be used for anti-inflammatory purposes. The gel is described as acting as a slow release H₂O₂ reservoir.

However, the use of UV-light to activate TiO₂ is not always convenient when it comes to products for medical and/or dental use.

It is a well-known fact that the morbidity and frequency of adverse effects, such as e.g. post-surgery effects, are directly related to, and often proportional to, the time used for the cleaning and/or debridement of surgically exposed hard tissue surfaces. Thus, rapid treatment ensures a better total treatment outcome. In addition, the total treatment outcome may also depend on the degree of damaging of the anatomical structure by the tool during the procedure. Furthermore, the total treatment outcome may also depend on the amount of contaminating material residues that is left on the treated surface by the tool.

Consequently, there is still sought for a means for cleaning and/or debriding implants and/or any hard tissue surface in the oral cavity, that guarantees rapid treatment without damaging the surface structure, and without leaving contaminating material residues, and which is antimicrobial and anti-inflammatory. The present invention for the first time presents such a means for effectively cleaning and/or debriding even difficult to access areas of rough hard surfaces in the oral cavity, and which does not need any secondary activation, such as by UV light radiation.

### Summary of the invention

The present invention relates to a new and inventive composition for implant cleaning and/or debridement of hard surfaces in the oral cavity, such as surgically exposed hard tissue surfaces, which comprises activated nanoparticles of TiO₂, having a mean particle diameter (D₅₀) of about 10-100 nm at a concentration between 0.5-500g/L, and H₂O₂, at a concentration of at the most 7.5% by volume, said composition being antibacterial, essentially without causing microbial resistance, and anti-inflammatory, and wherein said composition further comprises solid microparticles, having a mean particle diameter (D₅₀) of about 100-200 µm at a concentration between 0.5-300 g/L, for improved mechanical debridement and/or for improved cleaning of rough surfaces.

### Definitions

In the present context, the term "nanoparticle" is meant to describe a particle having a mean particle diameter (D₅₀) between about 1 and 1000 nm. Typically, in the present invention, a nanoparticles is used that has a mean particle diameter (D₅₀) between 10-100nm.

The term "microparticle" is herein meant to describe a particle having a mean particle diameter (D₅₀) between about 1 and 1000 µm. Typically, in the present invention, a microparticle is used that has a mean particle diameter (D₅₀) between 100-200*µ*m.

The present invention provides the means to clean and/or debride a medical and/or dental implant. In the present context, the term "implant" typically means a medical and/or dental implant that mainly comprises metal components. In general, though, an implant according to the present invention comprises at least one at least partially metallic surface.

In the present context, the term "dental implant" includes within its scope any device intended to be implanted into the oral cavity of a vertebrate animal, in particular a mammal such as a human, for example in tooth restoration procedures. Dental implants are herein selected from the group consisting of: Implants, bars, bridges, abutments, crowns, caps, and prosthetic parts in the oral cavity. Dental implants may also be denoted as dental prosthetic devices. Generally, a dental implant is composed of one or several implant parts. For instance, a dental implant usually comprises a dental fixture coupled to secondary implant parts, such as an abutment and/or a dental restoration such as a crown, bridge or denture. However, any device, such as a dental fixture, intended for implantation may alone be referred to as an implant even if other parts are to be connected thereto.

In the present context, the term "orthopedic implant" includes within its scope any device intended to be implanted into the body of a vertebrate animal, in particular a mammal such as a human, for preservation and restoration of the function of the musculoskeletal system, particularly joints and bones, including the alleviation of pain in these structures. Non-limiting examples of orthopedic implants are hip-joint prostheses, knee prostheses, elbow prostheses, finger prostheses, cochlear prostheses, and fixation screws.

In the present context, the term "vascular stent" refers to a tubular implant arranged for insertion into blood vessels of a vertebrate animal, in particular a mammal such as a human, in order to prevent or counteract a localized flow constriction, i.e. in order to counteract significant decreases in blood vessel diameter.

The term "a rough surface" is presently employed to describe grit blasted and/or etched titanium surfaces being mostly in the µm range, with a typical Ra range between 0.5 µm-10 µm. Typically, the rough surface is achieved by modifying a surface by machining, blasting, acid etching, or by a combination thereof.

In one embodiment of the present invention the term "rough surface" is meant to relate to an implant surface that is at least partially hydrophilic. This property can e.g. be achieved by a method in which, optionally after a preceding mechanical surface modification by material removal and/or chemical surface modification, at least the areas exposed of this surface exposed to bone and/or soft tissue are further chemically modified .

Hard tissues are, for example, bone, cementum, dentin, enamel, teeth, roots, cartilage and ligaments.

The term "debridement" means cleaning of a hard tissue surface, such as a surgically exposed hard tissue surface, in order to remove, for example, biofilm, concrements, microbes, unwanted tissue, cells and cell residues, scar tissue, and/or necrotic tissue. Debridement may, for example, be performed in order to control and/or treat local infections, inflammations, foreign body reactions, pathological conditions, and/or regenerative processes (e.g. periodontitis, periimplantitis).

### Figure Legends

Figure 1: SEM pictures of the three surfaces used in this study (x5000 in magnification).
Figure 2: SEM pictures of the three surfaces inoculated with *S.ep*. and incubated at 35°C for 16 hours. A biofilm covers all the surfaces (x5000 of magnification).
Figure 3: SEM pictures of the samples washed with NaCl for the control group or with H2O2+TiO2 for the test group. The biofilm is partially removed from the surfaces of the test group (x5000 of magnification).
**Figure 4****:** Photospectrometry absorbance results assessing the safranin concentration from the SLActive surfaces (n=9, * if p-value < 0.05).
**Figure 5****:** Photospectrometry absorbance results assessing the safranin concentration from the TiUnite surfaces (n=9, * if p-value < 0.05).
**Figure 6****:** Photospectrometry absorbance results assessing the safranin concentration from the OsseoSpeed surfaces (n=9, * if p-value < 0.05).
**Figure 7****:** Photospectrometry absorbance results assessing the biofilm re-growth after exposure of the samples to NaCl and H2O2+TiO2. The re-incubation of the samples for 4 hours showed a significantly lower bacterial re-growth due to the anti-bacterial effect of the nano-suspension compared to NaCl (n=9, * if p-value < 0.05).

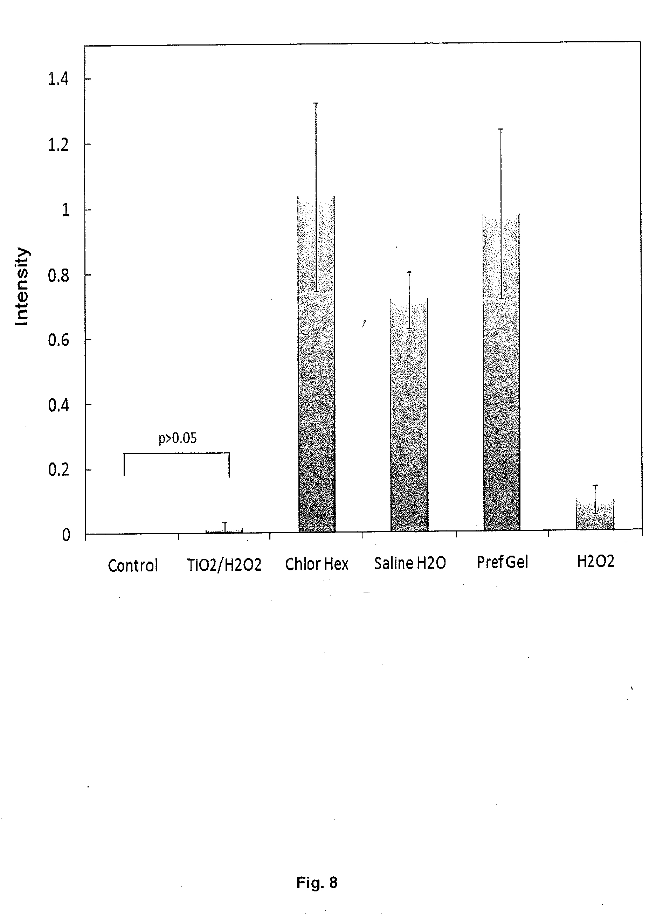

**Figure 8****:** Biomass assessed by measuring the absorbance intensity of the safranin staining from each surface exposed to the various chemical solutions. All groups were measured as statistically significant against each other (p<0.05), and the solution of the H2O2+TiO2 was removing most biofilm. Count index showing the presence of bacteria on the titanium surface (n=53).

### Detailed description of the invention

The present invention is directed to a composition comprising optimally activated nanoparticles of TiO₂, as well as H₂O₂ and microparticles.

The presently described composition is a long sought for means for cleaning and/or debriding an implant and/or any hard tissue surface in the oral cavity for rapid treatment without damaging the to be cleaned surface structure, and essentially without leaving contaminating material residues, at the same time displaying an antimicrobial and/or anti-inflammatory effect. The present invention thus for the first time presents a composition for effectively cleaning and/or debriding implants and/or any hard tissue surface in the oral cavity, including difficult to access areas on rough implant surfaces in the oral cavity, which does not need any secondary activation, such as by UV light radiation.

The present invention is therefore directed to an antimicrobial and/or anti-inflammatory composition for implant cleaning and/or debridement of a hard surface in the oral cavity, which comprises
a) nanoparticles of TiO₂, having a mean particle diameter (D₅₀) of 10-100 nm at a concentration between 0.5-500 g/L
b) H₂O₂ at a concentration of at the most 7.5% by volume, and
c) solid microparticles having a mean particle diameter (D₅₀) of 100-200 µm at a concentration between 0.5-300 g/L.

### Titanium particles

A ceramic is an inorganic, non-metallic solid, typically prepared by the action of heat and subsequent cooling.

Titanium dioxide is the naturally occurring oxide of titanium and has the chemical formula TiO₂. Titanium dioxide occurs in nature as well-known minerals rutile, anatase and brookite. The most common form is rutile. Rutile, anatase and brookite all contain six coordinated titanium. Rutile has a primitive tetragonal unit cell, with unit cell parameters a=4.584Å, and c=2.953Å. It therefore has a density of 4240 kg/m3.

Anatase has tetragonal shaped crystal structure. Although the degree of symmetry is the same for rutile and anatase, there is no relation between the interfacial angles of the two minerals, except, of course, in the prism-zone of 45° and 90°. The common pyramid of anatase, parallel to the faces of which there are perfect cleavages, has an angle over the polar edge of 82°9', the corresponding angle of rutile being 56°52½'.

Brookite has an orthorhombic shaped crystal structure.

Anatase and brookite both convert to rutile upon heating when heated above 915°C.

Preferably, the TiO₂ in the composition of the invention is predominantly in the anatase form. By predominantly is meant that at least 50% of the TiO₂ nanoparticles are in anatase form. More preferably, at least 60% of the TiO₂ nanoparticles are in the anatase form, even more preferably at least 70%, yet more preferably at least 80%. Also, at least 85%, such as 90 or 95%, of the TiO₂ may be in anatase form. The remainder of the TiO₂ may be in rutile and/or brookite form.

As mentioned above, it has previously been disclosed that TiO₂, when activated by UV-light, forms the free radical TiO-OH⁻. In the present invention, surprisingly, ceramic TiO₂ is activated to form the free radicals in the presence of H₂O₂ without necessitating any initial activation by UV-light. It was found that a specific ratio of surface exposure of TiO₂ nanoparticles to H₂O₂, facilitated by selecting a sufficient size of TiO₂ nanoparticles at a sufficient concentration will allow for optimal, i.e. spontaneous and feed-back activation of TiO₂ to form the free radicals on the surface of TiO₂. When the nanoparticles of TiO₂ are mixed with the H₂O₂ their surface is activated and radicals such as Ti-OH⁻, Ti- µ-peroxide and Ti-η²-peroxide are formed (Teruhisa O., et al. 2001). The composition of the invention may therefore also be denoted as a composition comprising activated nanoparticles of TiO₂, i.e. nanosized Ti-OH⁻, Ti- µ-peroxide and/or Ti-η²-peroxide particles, wherein "activated" means that the formation of the free radical at the surface of at least part of the TiO₂ nanoparticles has taken place.

A possible mechanism of a catalytic redox reaction on the surface of the TiO₂ nanoparticles is:

2TiO₂ + 2O₂⁻ + 2H⁺ → Ti₂O₃ + 2O₂ + H₂O (I)

2 TiO₂ + H₂O₂ → Ti₂O₃ + O₂ + H₂O (II)

Ti₂O₃ + OONO⁻→ 2 TiO₂ + NO₂⁻ (III)

Ti₂O₃ + H₂O₂ → 2 TiO₂ + H₂O (IV)

(See e.g. Suzuki R, et al., 2003 and Tengvall P, et al. 1989)

### The composition

The new and inventive composition for implant cleaning and/or debridement of hard surfaces in the oral cavity, presented herein, comprises nanoparticles of TiO₂, having a mean particle diameter (D₅₀) of about 10-100 nm at a concentration between 0.5-500 g/L TiO₂, and H₂O₂, at a concentration of at the most 7.5% by volume, as well as solid microparticles, having a mean particle diameter (D₅₀) of about 100-200 µm, at a concentration between 0.5-300 g/L.

The TiO₂ particles in the composition of the invention are of nanosize. The particles have a mean particle diameter (D₅₀) of about 100 nm or less. Preferably, the mean particle diameter is about 5-100 nm, such as 5-90 nm, 5-80 nm, 5-70 nm, 5-60 nm, 5-50 nm, 5-40 nm, 5-30 nm, 10-100 nm, 10-50 nm, 10-40 nm, 10-30 nm, 20-30 nm, or 15-25 nm. Presently preferred is a mean particle diameter of about 20-30 nm, such as selected from the group consisting of 20, 21, 22, 23, 24, 25, 26, 27, 28, 29 and 30 nm.

The selected range of size of the TiO₂ nanoparticles used in the present invention ensures that the particles are so small that they are easily pinocytozed by macrophages. However, the herein selected nanoparticles of TiO₂ are still not so small that they may penetrate cell walls and/or membranes on their own volition.

The concentration of TiO₂ nanoparticles in the composition of the invention is about at least 0.5 g/L. Preferably the concentration of TiO₂ nanoparticles is about 0.5-500 g/L, such as 0.5-10g/L, 0.5-5g/L, 0.5-4g/L, 10-300 g/L TiO₂, 15-250 g/L TiO₂, or about 50-200 g/L TiO₂. The concentration of TiO₂ nanoparticles in the composition of the invention may therefore be e.g. at least 0.5, 1, 2, 3, 4, 5, 10, 15, 20, 25, 50, 60, 70, 80, 90, 100, 200, 210, 220, 250 or 500 g/L. In one embodiment, the TiO₂ concentration is up to 4 g/L.

The present inventors found an increased anti-bacterial effect with a higher concentration of TiO₂ concentration up to 4 g/L. After this concentration, no increase in antibacterial effect was observed with more TiO₂ added. However, the composition of the invention may still comprise 0.5-500 g/L TiO₂ nanoparticles as a higher concentration of nanoparticles of TiO₂ will potentially aid the mechanical debridement. Also, it was surprisingly found that, if the TiO₂ concentration was held constant, there is an increased anti-bacterial effect with higher H₂O₂ concentration. However, for *in situ* use, the H₂O₂ concentration is limited to approximately at the most comprsising 7.5 Vol% of H₂O₂ in order not to affect biological tissue negatively.

The concentration of H₂O₂ in the composition of the invention is at the most about 7.5% by volume, preferably about at the most 3-7.5% by volume, such as 3, 4, 5, 6, 7% by volume.

One advantage with the use of the activated nanoparticles of TiO₂ (i.e. comprising the radicals as described above) is that after they have performed their task, e.g. killed microorganisms, they return to their less active state, i.e. unactivated TiO₂. This can e.g. be compared to silver ions that continue to be biologically active, which also means that they continue to be toxic to the environment as they are not inactivated after they have performed their tasks. The TiO₂ nanoparticles in the composition of the invention may however be reactivated to form the active radical species, e.g. by the careful application of UV-light Also the TiO₂ nanoparticles are anti-inflammatory even when they are not in their active state and may as such also have a lasting anti-inflammatory effect.

The composition described herein is antibacterial, unselectively and essentially without causing microbial resistance, and anti-inflammatory, and due to said composition, in addition to nanoparticles of TiO₂ and H₂O_{2,} further comprising solid microparticles, having a mean particle diameter (D₅₀) of about 80-250 µm at a concentration between 0.5-300 g/L, such as selected from the group consisting of 10, 15, 25, 50, 100, 200 and 300 g/L, it also displays improved mechanical means for debridement and/or cleaning of rough implant surfaces, which e.g. may have been modified by machining, blasting, acid etching, or by combinations of these procedures.

Therefore, the present invention provides compositions comprising solid microparticles, having a mean particle diameter (D₅₀) of about 80-250 µm, such as between 80-180, 120-180, 100-200, or between 100-150 µm. Preferably, the solid microparticles have a mean particle diameter (D₅₀) of about 120-180 µm, such as 120, 125, 130, 135, 140, 145, 150, 155, 160, 165, 170, 175, and 180 µm.

The concentration of solid microparticles in the composition of the invention is preferably 10-200 g/L , such as between 10-25, 10-50, 50-100, 50-150, 10-180, 100-150, 100-200, 150-200 or between 100-150 g/L, such as selected from the group consisting of approximately 10, 15, 25, 50, 80, 100, 120, 150, 180 and 200 g/L.

The microparticles are preferably biocompatible and solid (hard) and may also be biodegradable.

The solid microparticles may be selected from the group of material consisting of TiO₂, zirconium oxide, diamond dust (carbons), polymers, polylactic acid (beans), mineral, ceramic, dialuminium trioxide, calcium carbonate, calcium phosphate, apatite crystals, bone ceramic particles (hydroxyapatite/calcium phosphate), titanium, zirkonium, aluminium oxide, carborundum, pumice, and silica. The choice of material for the solid microparticles is preferably made depending on which material, e.g. a metal implant or a hard tissue surface, is to be cleaned/debrided by the composition of the invention, in order to fit the roughness of the material to allow for efficient cleaning/debriding of the material while still not damaging it.

Even in the case wherein the microparticles are made of TiO₂, the addition of microparticles to the composition of the invention will not essentially effect the optimized ratios between nanoparticles and H₂O₂ specified herein.

One advantage with the selection of the above specified size of the microparticles is that surface treatment of implants typically results in a diameter size of indents formed which is between 80-180 µm. Therefore, the presence of the solid microparticles in the composition of the invention makes the composition particularly suitable for the *in situ* cleaning and/or debridement of implants in the oral cavity as the microparticles are of a size that allows their entry into the indents to clean these, while still being large enough to not cause inflammatory reactions and/or to be encapsulated by the body in fibrous capsules.

A presently preferred composition of the invention comprises 5 Vol % H₂O₂ and 25 g/L nanoparticles (having a mean particles diameter (D₅₀) of 30 nm) and 20 g/L of solid macroparticles (having a mean particle diameter (D₅₀) of 100 um).

Another equally preferred composition of the invention comprises 4-5 Vol % H₂O₂ and 20-25 g/L nanoparticles (having a mean particles diameter (D₅₀) of 30 nm) and 15-20 g/L of solid macroparticles (having a mean particle diameter (D₅₀) of 100 um).

An antimicrobial and/or anti-inflammatory composition according to the present invention is typically formulated as a suspension of solid particles in a liquid.

In one embodiment, the antimicrobial and/or anti-inflammatory composition according to the present invention further comprises one or more emulsifier(s) and/or viscosity modifier(s). Said emulsifier and/or viscosity modifier may be selected from the group consisting of glycerine, glycols, polyethylene glycols (PEG), polyoxyethylene polyoxypropylene block copolymer (pluronic polyols), polyglycol alginate (PGA), CMC (carboxyl methyl cellulose), glycerol, Aloe Vera gel, alginate and citosan.

The antimicrobial and/or anti-inflammatory composition according to the present invention may comprise one or more detergent(s) selected from the group consisting of SDS (sodium dodecyl sulfate), sodium stannate, sodium pyrophosphate, oxine and SLS (sodium lauryl sulfate).

The antimicrobial and/or anti-inflammatory composition according to the invention may further comprise one or more flavouring oil(s), such as, but not limited to oils of spearmint, peppermint, wintergreen, sassafras, clove, sage, eucalyptus, marjoram, cinnamon and methyl salicylate and menthol.

In a preferred aspect, the composition of the invention consists of said nanoparticles of TiO₂, H₂O₂ and microparticles of the sizes and concentrations specified herein.

An antimicrobial and/or anti-inflammatory composition according to the present invention can be mixed before application and eventual storage, or stored separately and mixed directly or shortly before and/or at the time of application. The application therefore, in another aspect is directed to a kit comprising a first container comprising component a), a second container comprising component b), and a third container comprising component c), wherein a) comprises nanoparticles of TiO₂, having a mean particle diameter (D₅₀) of 10-100 nm at a concentration between 0.5-500 g/L, b) comprises H₂O₂ at a concentration of at the most 7.5% by volume, and c) comprises solid microparticles having a mean particle diameter (D₅₀) of 100-200 µm at a concentration between 0.5-300 g/L. Optionally such a kit may also comprise instructions for the preparation of the composition of the invention. The kit may also comprise one or more devices for the application of the composition to a subject. Such a device may e.g. be a syringe or an implant cleaning and/or debridement tool for cleaning and/or debriding an implant in the oral cavity. Preferably the implant cleaning and/or debridement tool comprises an elongated base member formed of at least two wires being twisted with each other, and a plurality of bristles fixed between said twisted wires and extending away from said twisted wires, whereby said bristles are positioned in a cleaning section at a first end of said base member; and that said bristles consist of titanium and/or a titanium alloy. A kit of the invention may also comprise the composition of the invention in one container and an implant cleaning and/or debridement tool for cleaning and/or debriding an implant in the oral cavity. One example of such an implant cleaning/debridement tool for cleaning a dental implant and/or debriding a hard tissue surface is disclosed in US 6,345,406, another example is given in WO 2009/083281. The kit may consist of a), a second container comprising component b), and a third container comprising component c). Also, the kit may consist of a), a second container comprising component b), and a third container comprising component c) and a implant cleaning/debridement tool for cleaning a dental implant and/or debriding a hard tissue surface. Alternatively, the kit may consist of a container consisting of the composition of the invention and an implant cleaning and/or debridement tool for cleaning and/or debriding an implant in the oral cavity.

The implant cleaning/debridement tool disclosed in WO 2009/083281 has bristles with diameters of 0.2 mm. The composition of the invention is particularly suitable to be used with this tool as the size of the solid microparticles in the composition will allow for an efficient cleaning of an implant and/or hard surface in the oral cavity. For this aspect, the microparticles optimally have a size about 150 µm, such as between 100 and 150µm, because the body tends to integrate particles in fibrous capsules when the particles are between 10-100 µm.

Periimplantitis is a typical complication related to orodental rehabilitation through the use of implants, i.e. a peri-implant disease, which is well-known to the person skilled in the art as an inflammatory reaction in which there is a loss of the bony support of the implant accompanied by inflammation. The aetiology of the disease is conditioned by the status of the tissue surrounding the implant, implant design, degree of roughness, the poor alignment of implant components, external morphology and excessive mechanical load.

The presently described antimicrobial and/or anti-inflammatory composition for the first time offers the means for an effective and rapid cleaning of an implant and/or for debriding a hard surface in the oral cavity essentially without damaging of the anatomical structure or of the implant and/or hard surface itself, and essentially without leaving contaminating material residues on the treated surface.

The invention therefore in one aspect is directed to the antimicrobial and/or anti-inflammatory composition as defined herein and/or the kit for preparing the composition of the invention as defined herein, for use as a medicament.

Thus, the present invention relates to the use of an antimicrobial and/or anti-inflammatory composition according to the present invention for cleaning and/or debriding an implant in the oral cavity, such as an implant *in situ*, a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect. The invention also relates to the use of the antimicrobial and/or anti-inflammatory composition as defined herein and/or the kit for preparing the composition of the invention as defined herein, for the preparation of a medicament and/or a pharmaceutical and/or cosmetic composition, for cleaning and/or debriding an implant in the oral cavity, such as an implant *in situ*, a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect. The invention is also directed to the antimicrobial and/or anti-inflammatory composition as defined herein or the kit for preparing the composition of the invention as defined herein for use for cleaning and/or debriding an implant in the oral cavity, such as an implant *in situ*, a hard surface in the oral cavity, such as an outer surface of a hard tissue in the oral cavity, a surgically exposed hard surface in the oral cavity, a wound in the oral cavity, such as a wound resulting from periimplantitis or a surgical wound, a periodontal defect and/or periodontal wound, and/or an oral hard tissue defect.

Another presently preferred embodiment is directed to the use of an antimicrobial and/or anti-inflammatory composition according to the present invention together with an implant cleaning and/or debridement tool, for cleaning an implant and/or debriding a hard surface in the oral cavity. Said implant cleaning and/or debridement tool is e.g. characterized by comprising an elongated base member formed of at least two wires being twisted with each other, and a plurality of bristles fixed between said twisted wires and extending away from said twisted wires, whereby said bristles are positioned in a cleaning section at a first end of said base member; and that said bristles comprise or consist of titanium and/or a titanium alloy.

Many medical implants, such as e.g. dental implants, orthopedic implants and vascular stents, are metallic, i.e. they are made of a metal material. The present invention consequently relates to the use of an antimicrobial and/or anti-inflammatory composition according to the present invention, alternatively together with an implant cleaning and/or debridement tool, for cleaning and/or debriding an implant made of a metal material. Examples of metal materials commonly utilized for constructing metallic medical implants are steel, titanium, zirconium, tantalum, niobium, hafnium and alloys thereof. In particular, titanium and titanium alloys have proven to be suitable to utilize for constructing medical implants.

On the other hand, both medical and dental implants can at least partially, as well as in full (full-ceramic implants) consist of porcelain and/or ceramic, such as of zirconium oxide and/or hydroxyapatite, or any other ceramic or porcelain material known to the person skilled in the art as being suitable for implantry. Thus, the present invention equally relates to the use of an antimicrobial and/or anti-inflammatory composition according to the present invention, alternatively together with an implant cleaning and/or debridement tool, for cleaning and/or debriding an implant made of, or comprising porcelain and/or ceramic. Consequently, the present invention is also directed to the use of a composition of the invention for the preparation of a medicament and/or a pharmaceutical and/or cosmetic composition for the cleaning and/or debridement of an implant made of or comprising porcelain and/or ceramic. Also, the invention is directed to a composition of the invention alternatively for use for cleaning and/or debriding an implant made of or comprising porcelain and/or ceramic.

Dental implants are typically utilized in dental restoration procedures in patients having lost one or more of their teeth. A dental implant comprises a dental fixture, which is utilized as an artificial tooth root replacement. Thus, the dental fixture serves as a root for a new tooth. The dental fixture is typically a screw, i.e. it has the shape of a screw, and it is typically made of titanium, a titanium alloy, zirconium or a zirconium alloy. The screw is surgically implanted into the jawbone, where after the bone tissue grows around the screw and the screw is fixated in the bone with the bone in close contact with the implant surface. Once the implant screw is firmly anchored in the jawbone, it may be elongated by attachment of an abutment to the screw. The abutment may, just as the screw, be made of titanium, a titanium alloy, zirconium or a zirconium alloy. The shape and size of the utilized abutment are adjusted such that it precisely reaches up through the mucosa after attachment to the screw. A dental restoration such as a crown, bridge or denture may then be attached to the abutment. Alternatively, the implant screw has such a shape and size that it reaches up through the mucosa after implantation, whereby no abutment is needed and a dental restoration such as a crown, bridge or denture may be attached directly to the screw.

The present invention consequently relates to the use of an antimicrobial and/or anti-inflammatory composition according to the present invention, alternatively together with an implant cleaning and/or debridement tool, for cleaning and/or debriding any parts of a dental implant, selected from the group consisting of dental fixture such as a screw, abutment, and dental restoration such as a crown, bridge or denture. Consequently, the present invention is also directed to the use of a composition of the invention for the preparation of a medicament and/or pharmaceutical and/or cosmetic composition for cleaning and/or debriding any parts of a dental implant, selected from the group consisting of dental fixture such as a screw, abutment, and dental restoration such as a crown, bridge or denture. Also, the invention is directed to a composition of the invention for use for cleaning and/or debriding any parts of a dental implant, selected from the group consisting of dental fixture such as a screw, abutment, and dental restoration such as a crown, bridge or denture.

The present invention further relates to the use of an antimicrobial and/or anti-inflammatory composition according to the present invention, alternatively together with an implant cleaning and/or debridement tool, for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents, i.e. tubular implants arranged for insertion into blood vessels in order to prevent or counteract a localized flow constriction. Consequently, the present invention is also directed to the use of a composition of the invention for the preparation of a medicament for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents. Also, the invention is directed to a composition of the invention for use for cleaning and/or debriding orthopaedic implants, such as orthopaedic implants which are utilized for the preservation and restoration of the function in the musculoskeletal system, particularly joints and bones, including alleviation of pain in these structures, and/or for cleaning and/or debriding vascular stents.

The surface of medical implants such as e.g. dental implants, orthopedic implants and vascular stents, or the vicinity thereof, has sometimes to be cleaned after placing. This is particularly important when an infection or contamination occurs, causing a progressive degenerative process in the bone adjacent to the implant known as periimplantitis. In these cases the surface of the ailing implant has to be cleaned from microbes and contaminants to stop the progression of the disease and ensure re-integration of the implant. Failure to clean the implant surface will eventually lead to loss of bone and implant, and make further alternative treatments difficult and sometimes even impossible. Furthermore, the surface of vascular stents may have to be cleaned during implantation in order to remove coagulum, and the interior of vascular stents, i.e. the cavity within vascular stents, may have to be cleaned in an endoscopic procedure during a later treatment due to restenosis, i.e. blocking of the blood vessel.

The present invention therefore relates to the use of an antimicrobial and/or anti-inflammatory composition according to the present invention, alternatively together with an implant cleaning and/or debridement tool, for cleaning and/or debriding an implant or the vicinity thereof after placing. Consequently, the present invention is also directed to the use of a composition of the invention for the preparation of a medicament for cleaning and/or debriding an implant or the vicinity thereof after placing. Also, the invention is directed to a composition of the invention for use for cleaning and/or debriding an implant or the vicinity thereof after placing.

In addition, for different reasons, it may be advantageous or necessary to debride surgically exposed hard tissue surfaces. For example, debriding of surgically exposed hard tissue surfaces may be advantageous or necessary to perform before regenerative treatment, i.e. in order to prepare the hard tissue surfaces for regenerative treatment. Examples of conditions, which may be associated with a treatment in which debridement of a surgically exposed hard tissue surface is advantageous or necessary to perform in order to prepare the surface for regenerative treatment, are: periimplantitis, periodontitis lesions, marginal periodontitis, apical periodontitis, furcation defects, apical granulomas and cysts, bone cysts, bone tumors, bone granulomas, bone cancers, (infected) extraction sockets, alveolitis sicca ("dry socket"), cleaning of apicectomy defects, localized osteomyelitis, trauma induced defects, resection or revision of implants, resection or revision of fractures, and removal of temporary bone implants (such as orthopaedic bone plates, retainers and screws). Furthermore, debridement of articular surfaces in joints affected by arthritis and debridement of such surfaces before regenerative treatment for cartilage and ligaments is instituted may also be advantageous or necessary to perform.

The present invention thus relates to the use of an antimicrobial and/or anti-inflammatory composition according to the present invention, alternatively together with an implant cleaning and/or debridement tool, for cleaning and/or debriding surgically exposed hard tissue surfaces before regenerative treatment. Consequently, the present invention is also directed to the use of a composition of the invention for the preparation of a medicament and/or a pharmaceutical and/or cosmetic composition for cleaning and/or debriding surgically exposed hard tissue surfaces before regenerative treatment. Also, the invention is directed to a composition of the invention for use for cleaning and/or debriding surgically exposed hard tissue surfaces before regenerative treatment.

The antimicrobial and/or anti-inflammatory composition according to the present invention, alternatively together with an implant cleaning and/or debridement tool, may be utilized during surgery for cleaning of the surface of a metallic medical implant after infection and/or bone resorption. For example, it may be utilized for cleaning the surface of a metallic dental implant and/or a metallic orthopedic implant. Thus, it may be utilized for removing e.g. bacterial biofilm, debris, calculus or fibrous tissue from the surface of a dental implant, such as a titanium screw. Alternatively, it may be utilized together with a further cleaning agent (i.e. an antibacterial agent) in order to remove the bacterial biofilm from the vicinity of the dental fixture during implantation. It may also be utilized for cleaning the surface of, or the vicinity of, an abutment. Consequently, the present invention is also directed to the use of a composition of the invention for the preparation of a medicament for cleaning, e.g. removing bacterial biofilm, debris, calculus or fibrous tissue from the surface of a metallic dental implant, such as a titanium screw or an abutment, or a metallic orthopedic implant. Also, the invention is directed to a composition of the invention for use for cleaning, e.g. removing bacterial biofilm, debris, calculus or fibrous tissue the surface of a metallic dental implant, such as a titanium screw or an abutment, or a metallic orthopedic implant.

In addition, the antimicrobial and/or anti-inflammatory composition according to the present invention, alternatively together with an implant cleaning and/or debridement tool, may be utilized for removing cement remnants, bacterial biofilm, debris, calculus or fibrous tissue from the surface of an orthopedic implant or for removing plaque from the surface of a vascular stent. Alternatively, it may be utilized for cleaning the interior of a vascular stent, i.e. the cavity within a vascular stent, in an endoscopic procedure during a later treatment due to restenosis, i.e. blocking of the blood vessel.

A procedure involving use of the antimicrobial and/or anti-inflammatory composition according to the present invention, alternatively together with an implant cleaning and/or debridement tool, may, for example, involve the steps of: surgically exposing a hard tissue surface to be treated; removal of inflamed soft tissue; debriding the surface by means of applying the antimicrobial and/or anti-inflammatory composition according to the present invention, alternatively together with an implant cleaning and/or debridement tool; applying (regenerative) treatment as needed; replacing soft tissue; suturing for good primary closure and wound stability; and allowing the wound to heal.

In particular, the antimicrobial and/or anti-inflammatory composition according to the present invention, alternatively together with an implant cleaning and/or debridement tool, is an efficient tool for debridement of surgically exposed tooth root surfaces, furcation defects and bony defects before regenerative treatment (i.e. by means of, for example Straumann^{®} Emdogain, bone graft materials, autologous bone, membranes, etc.). the antimicrobial and/or anti-inflammatory composition according to the present invention, alternatively together with an implant cleaning and/or debridement tool, is especially effective for removing granulation tissue, and for removing concrements of calcified biofilms (plaques) and subgingival calcus.

The antimicrobial and/or anti-inflammatory composition according to the present invention, alternatively together with an implant cleaning and/or debridement tool, is advantageous to utilize for cleaning and/or debriding both "hard" metallic medical and/or dental implants having relatively hard surfaces, such as e.g. medical implants of steel, and "soft" metallic medical implants having delicate surfaces, such as e.g. medical and/or dental implants of titanium, a titanium alloy, zirconium or a zirconium alloy.

In addition, the antimicrobial and/or anti-inflammatory composition according to the present invention does not leave contaminants, i.e. material residues, incompatible with reintegration of the implanted structure. The use of nanoparticles of TiO₂, having a mean particle diameter (D₅₀) of 10-100 nm facilitates a biological removal of any material residues left after treatment, as these particles are small enough for the patient's macrophages for pinocytosis, while still being large enough to circumvent cell wall penetration. What is more, since titanium in itself is biocompatible, a foreign body response is usually not triggered. Thus, the inflammation risk is minimal.

In particular, a relatively rapid debridement procedure of surfaces, which are otherwise hard to clean and/or hard to reach by hand instrumentation, may be performed by means of the antimicrobial and/or anti-inflammatory composition according to the present invention, alternatively together with an implant cleaning and/or debridement tool. Rapid treatment ensures a better treatment outcome. As mentioned above, it is a well-known fact that the morbidity and frequency of adverse effects, such as e.g. post-surgery effects, are directly related to, and often proportional to, the time used for the debridement of surgically exposed hard tissue surfaces. Thus, rapid debridement treatment ensures a better total treatment outcome.

The use of the antimicrobial and/or anti-inflammatory composition according to the present invention, alternatively together with an implant cleaning and/or debridement tool, is especially favorable where the treatment plan for a defect includes placing of a titanium implant or any other device made of titanium, since only titanium and no other metallic ions or polymers that can provoke unwanted and/or adverse clinical and/or biological effects can contaminate the treated area, hampering the outcome of planned and/or future implant procedures.

### Oral hygiene

In oral hygiene and dentistry, debridement refers to the removal of plaque and calculus that have accumulated on the teeth, which can be performed routinely by the technician, for medical, hygienic, as well as for purely cosmetic reasons. Thus, in one embodiment, the antimicrobial and/or anti-inflammatory composition according to the present invention, again alternatively together with an implant cleaning and/or debridement tool, is used for removal of plaque and calculus that have accumulated on the patient's natural teeth, or tooth implants. The antimicrobial and/or anti-inflammatory composition according to the present invention comprises radicalized oxygens, and is thus particularly suitable for use in the bleaching of natural and/or artificial teeth.

### Microorganisms

The microorganisms most commonly associated with implant failure are spirochetes and mobile forms of Gram-negative anaerobes. Diagnosis can be based on changes of colour in the gum, bleeding and probing depth of peri-implant pockets, suppuration, x-ray and gradual loss of bone height around the tooth. The antibiotic therapy proven to be most efficacious in the antibiogram has so far been the association of amoxycillin and clavulanic acid. Additionally to bacterial infections, microbial infections in the oral cavity can of course also include fungal and/or viral infections.

An antimicrobial and/or anti-inflammatory composition according to the present invention is effective for killing bacteria, fungus and/or virus.

What is more, the composition described herein is antimicrobial, without causing microbial resistance, as well as anti-inflammatory. This is at least in part due to the fact that the radicals from the TiO₂ surface attack the bacterial membrane. Thus, the radicals kill essentially any type of bacteria and the bacteria can thus not become resistant to this compound, in comparison to traditional antibiotics, which are more strain specific. Furthermore, once the radicals have killed the bacteria, they return to their original form TiO₂.

Compared to other antimicrobial agents, TiO₂ is particularly suitable for use in the oral cavity and of implant surfaces, due to properties such as stability, environmental safety, broad spectrum antibiosis and anti-inflammatory properties. Moreover, the TiO₂ free radicals actively modulate immune responses, acribate macrophages and stimulate the healing process. This means that they do not just kill microorganisms, but stimulate the surgical wound in the healing process as well. Thus, the optimally activated nanosized TiO₂ particles are particularly suitable for the next generation of bioactive antibacterial materials.

### Experimental section

### Example 1

### Paste for titanium surface debridement:

A suspension was prepared by adding H₂O₂ (PERDROGEN® 30% H₂O₂ (w/w), Sigma Aldritch AS, Oslo, Norway) at 5 vol %, 10 g/L of nanoparticles of TiO₂ (Aeroxide P25, Evonik AG, Essen, Germany) and 20 g/L of microparticles of TiO₂ (Hombitan, Kronos Titan Worldwide Inc, USA). The mean particle diameter (D₅₀) was measured by laser (Mastersizer 2000, Malvern, Herrenberg, Germany) and the distribution showed a large peak with D₅₀ of 40 nm and a second large peak at 100 µm.

### Example 2

### Controlling viscosity paste for titanium surface debridement:

A suspension was prepared as described in Example 1. The viscosity was altered by adding additionally 300 g/L of nanoparticles of TiO₂ (Aeroxide P25, Evonik AG, Essen, Germany) and 1000 g/L of microparticles of TiO₂ (Hombitan, Kronos Titan Worldwide Inc, USA). The suspension behaved like thick slurry.

### Example 3

### Controlling viscosity paste for titanium surface debridement:

A suspension was prepared as described in Example 1. The viscosity was altered by adding 30 grams of a gelling agent polyoxyethylene polyoxypropylene block copolymer (Pluronic® F-127, Sigma Aldritch, Oslo, Norway). The suspension behaved like thick slurry.

### Example 4

### Debridement of titanium surface:

A suspension was prepared as described in Example 1. Grit-blasted titanium surface with Sa value of 2 µm where contaminated with porphyromonas gingivalis. One group (n=6) was left untreated, and the other groups (n=6) where cleaned with a titanium bristle device, TiBrush™, with saline water, EDTA, 3 vol % H₂O₂ and the suspension as described in example 1. Bacteria count after the cleaning showed significant reduction in bacteria numbers for the groups cleaned with the suspension as described in example 1 when compared to all other groups.

### Example 5

### Anti-bacterial effect of activated TiO₂ nanoparticles on aerobe peri-implantitis-associated bacteria:

TiO₂ grit-blasted titanium coins (Grade IV) with diameter of 6.2 mm will be contaminated by aerobe peri-implantitis-associated bacteria (*Streptococcus mutans, S. sanguis, Actinomyces naeslundii*). The surface will be cleaned with titanium bristle device, TiBrush™ for 3 minutes with the following cleaning solutions (EDTA, saline water, 3%vol H₂O₂ and the suspension as described in example 1.). The coins will be individually placed in 1.5 ml Eppendorf tubes containing 500µl of cell culture medium (without antibiotics) of from Invitrogen (GIBSCO MEM, Invitrogen, Carlsbad, CA, USA). All the Eppendorf tubes containing the coins and the bacteria will be placed in an incubator, in the dark, at 37°C for 20 hours. After 20 hours, all the samples will be taken out of the incubator. A Spectrometer (Perkin Elmer UV-Vis 200, Oslo, Norway) will be calibrated with only 700µl of cell media for the base line. Then, the three Eppendorf tubes containing only 500µl of cell media + 10µl of the stock solution will be analyzed. Then, one by one the test tubes will be shaken and a volume of 400µl from each tube will be mixed with 300µl of cell media. The 1.5ml cuvettes contained 700µl of liquid to be analyzed.

### Example 6

### Anti-bacterial effect of TiO₂ nano-suspension

Removal of multilayer *Staphylococcus epidermidis* from three types of rough surfaces.

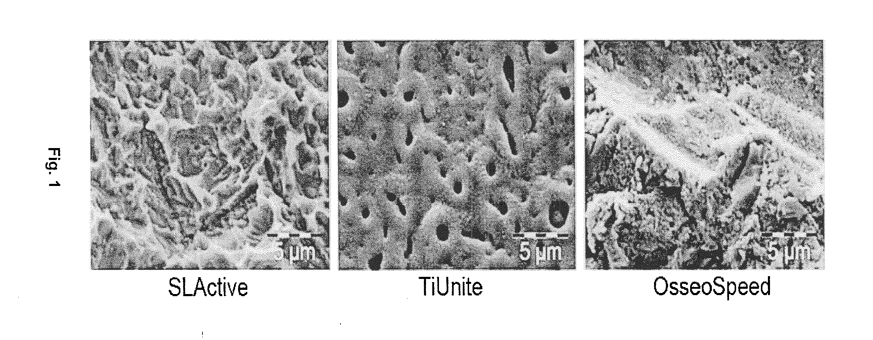

Coin-shaped titanium implants (grade 2, 6.2 in diameter and 2 mm in height) were used in these experiments. The three groups selected correspond to commercial implant surfaces: SLActive (Straumann, Basel, Switzerland), TiUnite (NobelBiocare, Zurich, Switzerland), OsseoSpeed (AstraTech, Mölndal, Sweeden). They all have a specific topography (fig 1).

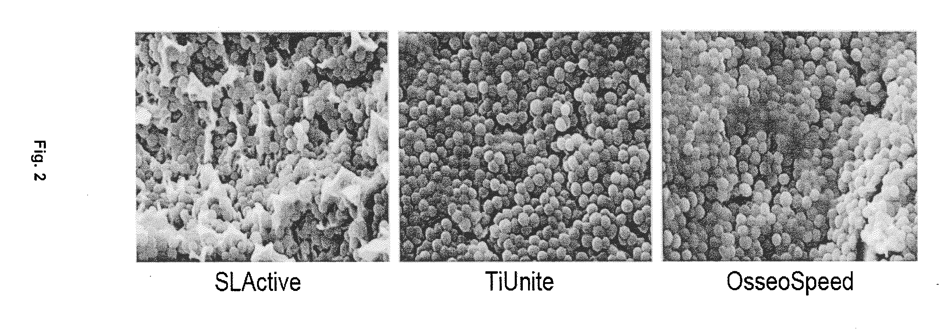

These samples were inoculated with *Staphylococcus epidermidis* (*S.ep.*) in culture medium (Brain Heart Infusion or BHI) and left in the incubator at 35°C for 16 hours. After the incubation period reached, all the samples were covered by a biofilm (fig 2). The samples were gently rinsed three times for 2 minutes with NaCl, then exposed to NaCl (control group) or to the nano-suspension (H₂O₂+ TiO₂ prepared as described in example 1) (test group). The samples were again rinsed and analyzed using two methods in order to detect the biomass still attaching to the surfaces.

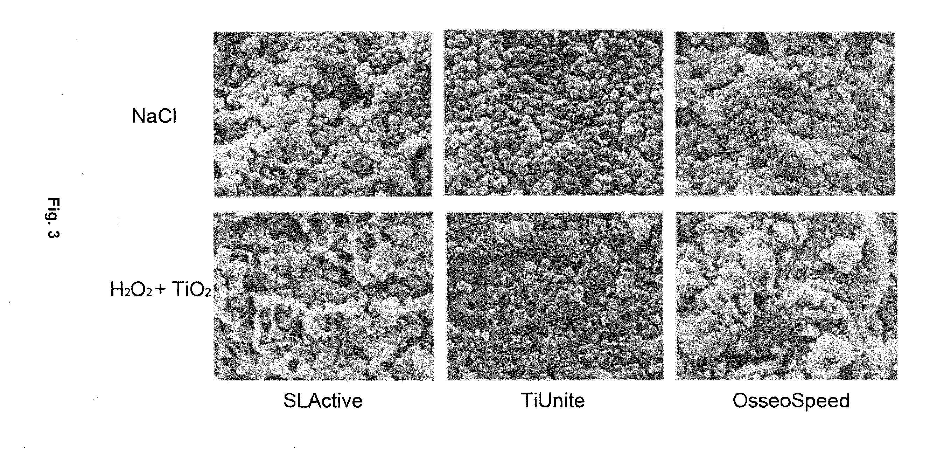

The first method employed was using a SEM at x5000 of magnification (fig 3).

By this visual and qualitative method, it is possible to detect a reduction of the biofilm density from the test group compared to the control group.

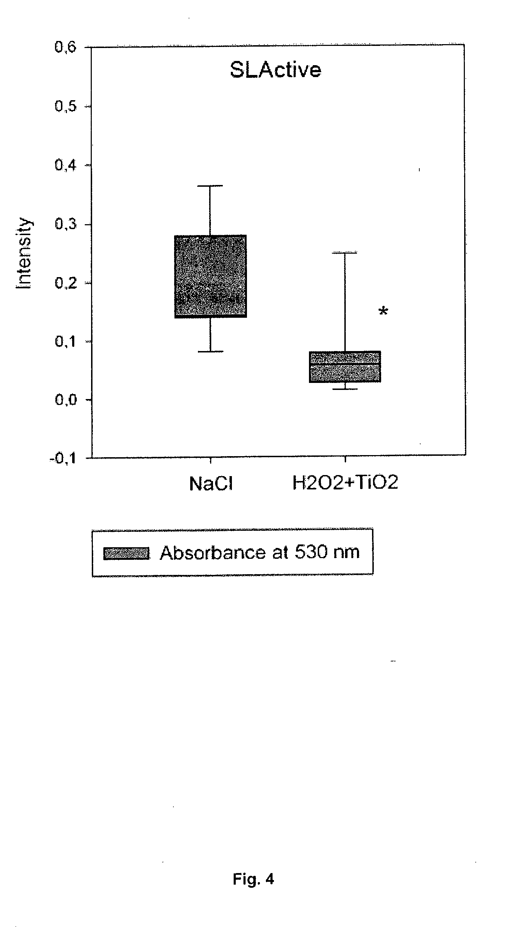

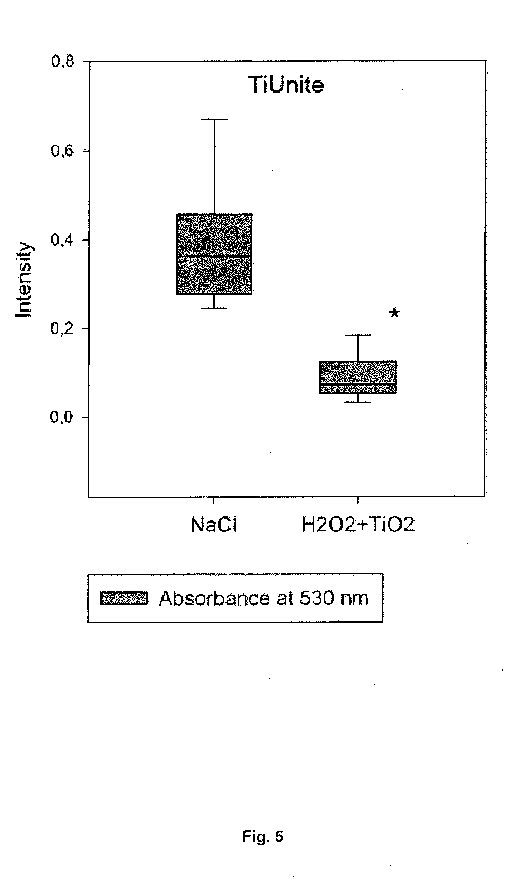

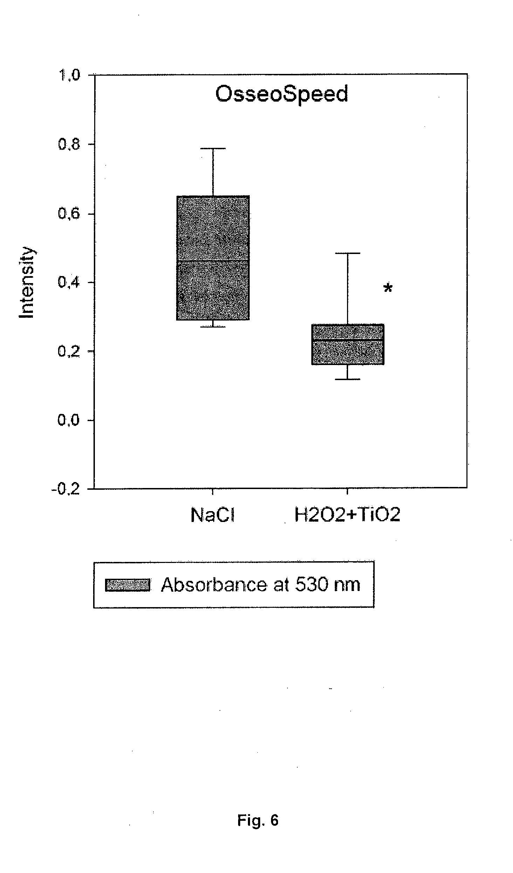

The second method used was by safranin staining the nucleus of the bacteria attaching on the surfaces after exposure to the solution. The staining pink color is then released from the surfaces and analyzed by using a spectrophotometer (Synergy HT Multi-Detection Microplate Reader, Biotek, Winooski, VT, USA) at a wavelength of 530 nm. This method was performed on each samples (n=9) of each group (fig 4, 5 and 6). A statistical analysis was conducted to determine if the data collected were statically significant (noted * if p-values<0.05).

From these results we could conclude that the amount of bacteria present on the surface of the samples was significantly lower after exposure to the nano-suspension (H₂O₂+TiO₂) compared to NaCl, no matter the topography.

Safranin staining gives quantitative results regarding the chemical potential alone in removing a biofilm formed after 16 hours of incubation. However, it is not possible to detect whether or not these bacteria are dead or not. Therefore, in order to test if this chemical disinfection could stop the biofilm growth, an other test was conducted in order to determine the viability of the biofilm after the disinfection.

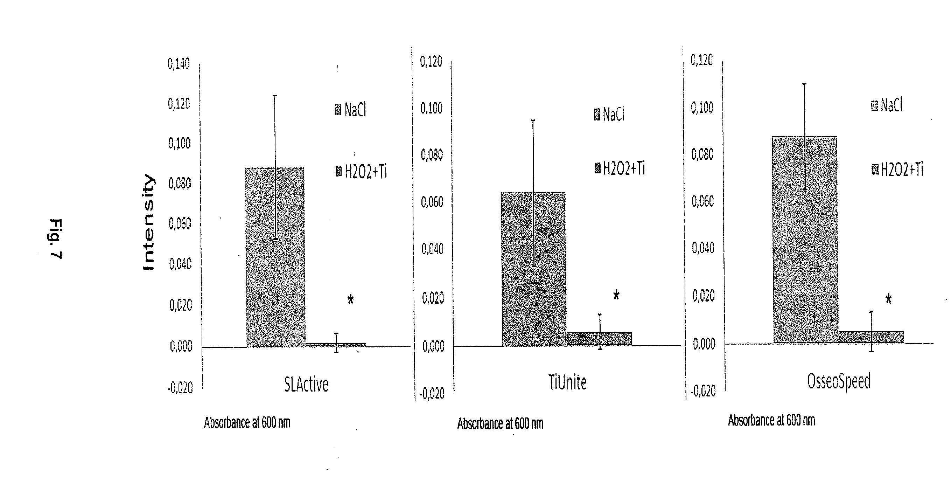

The method was similar to the safranin staining analysis, from the inoculation to the disinfection using both products (NaCl and nano-suspension). But this time, after the disinfection step, the samples were rinsed in NaCl and re-incubated at 35°C for four hours in pure BHI medium. The medium was then collected and analyzed using the same spectrophotometer but this time at a wavelength of 600nm. The intensity of the absorbance was compared between control and test groups (fig 7).
The results from this experiment lead to the conclusion that the nano-suspension of H₂O₂+TiO₂ decreases the bacterial re-growth compared to NaCl solution.

### Overall conclusion:

The nano-suspension of H₂O₂+TiO₂ decreases significantly the biomass presence from various commercialized dental implant surfaces as well as the bacterial re-growth compared to NaCl solution.

### Example 7

Titanium disks preparation and surface modification chemically pure (cp) titanium disks (n=53) with a diameter of 6.2 mm and a height of 2 mm were grinded and polished (Phoenix 4000, Buehler GmbH, Duesseldorf, Germany) in seven sequences. Silicon carbide papers from P800 to P4000, a porous neoprene for final polishing as well as the abrasive colloidal silica suspension (OP-S) were supplied by the same manufacturer (Struers GmbH, Willich Germany).
After polishing, the disks were washed with NaOH at 40 vol.% and HNO3 at 50 vol.% in an ultrasonic bath to remove contaminants, then washed with deionized water to reach a neutral pH, and stored at room temperature in 70 vol.% ethanol. Thereafter, the coins were placed in Eppendorf tube and steam autoclaved for sterilization.
Three chemical decontamination agents were selected for the in vitro testing: sterile saline H₂O (VWR, Oslo, Norway), Pref Gel, (Straumann Institut, Basel, Switzerland).0.2 vol% Chlorhexidine, 3 vol% H₂O₂ (VWR, Oslo, Norway) and a mixture of 3 vol% H₂O₂ and 2 g/L TiO2 (1g nanoparticles: P25 Aeroxide, Degussa Evonik, Evonik Industries AG, Essen, Germany + 1 g microparticles: Kronos 1171, Kronos Titan GmbH, Leverkusen, Germany).

In vitro testing: biofilm assay 53 polished and sterile titanium disks per groups were inoculated. The control group was inoculated with brain heart infusion broth (BHI) only, while the test groups (five) were inoculated with the bacteria culture (10 *µ*l Staphylococcus epidermidis + 5 ml BHI). The incubation time lasted for 24 h at 37°C in an aerobic atmosphere. The discs were then transferred to new wells, rinsed with sterile saline water, then were exposed to the five selected chemical agents for two minutes, then rinse again with sterile saline water. The amount of biofilm present on the surface of the titanium samples was assessed by using the safranin staining method: 10 min exposure to a 0.1 % solution of safranin, then rinsed with distilled water, air dried, and exposed to a solution of 30% acetic acid to release the colored biomass from the titanium surfaces. The intensity of the staining was analyzed using a Synergy HT Multi-Detection Microplate Reader (Biotek, VT, USA) with a wavelength of 530 nm.

### Statistical Analysis

A power analysis was performed on pilot data in order to find appropriate number of samples (SPSS 17.0 for Windows). One way ANOVA was used to compare all the groups. If the data set failed both equality and normality test and could not be normalized by transformation, non-parametric Kruskal-Wallis analysis of variance (ANOVA) on ranks was used instead. Significance differences between each groups (p-values < 0.05) was found using the Dunn's test (SigmaPlot 11.0, Systat Inc, St-Louis, USA).

### Results

Optical density analysis in the Synergy HT Multi Detection microplate Reader revealed that all the samples exposed to the chemical solutions had a statistically significant (p<0.05) increase of the biomass except for the group exposed to the 3 vol% H₂O₂ and 2g/L TiO₂ which was not significantly different than the control group (p>0.05).
All the groups were statistically significant between each others.
The suspension composed by a mixture of 3 vol% H₂O₂ and 2g/L TiO₂ (1g nanoparticles + 1 g microparticles) was the most effective in removing the biofilm from the contaminated titanium surfaces, significantly more effective than 3% H₂O₂ alone.

### References

1. US 6,345,406
2. WO 2009/083281
3. Yu Q, Amirsardari Y, Yu Q, Willams P. Environ Technol. 2001 Sep;22(9):1015-23.
4. Sunada K, Watanabe T, Hashimoto K. Environ Sci Technol. 2003 Oct 5;37 (20): 4785-9.
5. Byrne MF, Murray FE. Ir Med J. 1998 Jan-Feb;91(1):8-10. Review.
6. Maness PC, Smolinski S, Blake DM, Huang Z, Wolfrum EJ, Jacoby WA. Appl Environ Microbiol. 1999 Sep;65(9):4094-8.
7. Silva et al. 2007 "Effect of key operating parameters on phenols degradation during H2O2-assisted TiO2 photocatalytic treatment of simulated and actual olive mill wastewaters" Appl Catalysis 73 11-22
8. Teruhisa Ohno, Yuji Masaki, Seiko Hirayama, Michio Matsumura, TiO2-Photocatalyzed Epoxidation of 1-Decene by H2O2 under Visible Light, Journal of Catalysis, Volume 204, Issue 1, (2001), P. 163-168
9. Suzuki R, Muyco J, McKittrick J, Frangos JA. Reactive oxygen species inhibited by titanium oxide coatings. J Biomed Mater Res A 2003;66(2):396-402
10. Tengvall P, Lundstrom I, Sjoqvist L, Elwing H, Bjursten LM. Titanium- hydrogen peroxide interaction: model studies of the influence of the inflammatory response on titanium implants. Biomaterials 1989;10(3):166-75
11. WO 98/06548

## Claims

1. An antimicrobial and/or anti-inflammatory composition for implant cleaning and/or debridement of a hard surface in the oral cavity, which comprises
a. nanoparticles of TiO₂, having a mean particle diameter (D₅₀) of 10-100 nm at a concentration between 0.5-500 g/L
b. H₂O₂ at a concentration of at the most 7.5% by volume, and
c. solid microparticles having a mean particle diameter (D₅₀) of 100-200 µm at a concentration between about 0.5-300 g/L.

2. An antimicrobial and/or anti-inflammatory composition according to claim 1, wherein said nanoparticles of TiO₂ are selected from the group consisting of particles having a mean particle diameter (D₅₀) of 20-30 nm.

3. An antimicrobial and/or anti-inflammatory composition according to any one of the preceding claims, wherein said nanoparticles of TiO₂ are present at a concentration of about 10-300 g/L.

4. An antimicrobial and/or anti-inflammatory composition according to any of the preceding claims, wherein said nanoparticles of TiO₂ are crystalline.

5. An antimicrobial and/or anti-inflammatory composition according to any one of the preceding claims, wherein said solid microparticles are selected from the group consisting of particles having a mean particle diameter (D₅₀) of 120-180 µm.

6. An antimicrobial and/or anti-inflammatory composition according to any of the preceding claims, which comprises said solid microparticles at a concentration between 0.5-20 g/L.

7. An antimicrobial and/or anti-inflammatory composition according to any one of claims 1-6, wherein said solid microparticles are selected from the group consisting of TiO₂, zirconiumoxide, diamond dust, polymers, polylactic acid, mineral, ceramic, dialuminium trioxide, calcium carbonate, calcium phosphate, apatite crystals, bone ceramic particles, titanium, zirconium, aluminium oxide, carborundum, pumice, silica and mixtures thereof.

8. An antimicrobial and/or anti-inflammatory composition according to any one of the preceding claims, which comprises said H₂O₂ at a concentration of 3-7.5% by volume.

9. An antimicrobial and/or anti-inflammatory composition according to any one of the preceding claims, which further comprises one or more detergent(s) selected from the group consisting of SDS (sodium dodecyl sulphate) and SLS (sodium lauryl sulphate).

10. An antimicrobial and/or anti-inflammatory composition according to any one of the preceding claims, wherein said components a), b) and c) are mixed before application and eventual storage.

11. An antimicrobial and/or anti-inflammatory composition according to any one of claims 1-9, wherein at least one of said components a), b) and c) are stored separately and the composition is mixed directly before and/or at the time of application.

12. A kit comprising a first container comprising component a) as defined in any one of claims 1-4, a second container comprising component b) as defined in any one of claims 1 and 8, and a third container comprising component c) as defined in any one of claims 1 and 5-7, optionally together with instructions for the preparation of a composition as defined in any one of claims 1-11,
said kit optionally further comprising one or more devices for the application of the composition to a subject.

13. A kit according to claim 12, wherein the device for application of the composition is an implant cleaning and/or debridement tool for cleaning and/or debriding an implant in the oral cavity,
said implant cleaning and/or debridement tool comprising
a. an elongated base member formed of at least two wires being twisted with each other, and
b. a plurality of bristles fixed between said twisted wires and extending away from said twisted wires, whereby said bristles are positioned in a cleaning section at a first end of said base member; and that said bristles consist of titanium or a titanium alloy.

14. An antimicrobial and/or anti-inflammatory composition according to any one of claims 1-11 for use as a medicament.

15. An antimicrobial and/or anti-inflammatory composition according to any one of claims 1-11 for use for cleaning and/or debriding an implant in the oral cavity, a hard surface in the oral cavity, a wound in the oral cavity, a periodontal defect and/or periodontal wound, and/or an oral hard tissue defect.

## Patentansprüche

1. Antimikrobiotische und/oder antiinflammatorische Zusammensetzung für die Implantatreinigung und/oder das Debridement einer harten Oberfläche in der Mundhöhle, die umfasst
a. Nanopartikel von TiO₂, die einen mittleren Partikeldurchmesser (D₅₀) von 10-100 nm haben, bei einer Konzentration zwischen 0,5-500 g/l
b. H₂O₂ bei einer Konzentration von höchstens 7,5 Volumen-% und
c. feste Mikropartikel, die einen mittleren Partikeldurchmesser (D₅₀) von 100-200 µm haben, bei einer Konzentration zwischen etwa 0,5-300 g/l.

2. Antimikrobiotische und/oder antiinflammatorische Zusammensetzung gemäß Anspruch 1, wobei die Nanopartikel aus TiO₂ ausgewählt sind aus der Gruppe, bestehend aus Partikeln, die einen mittleren Partikeldurchmesser (D₅₀) von 20-30 nm haben.

3. Antimikrobiotische und/oder antiinflammatorische Zusammensetzung gemäß einem der vorangehenden Ansprüche, wobei die Nanopartikel aus TiO₂ in einer Konzentration von etwa 10-300 g/l vorliegen.

4. Antimikrobiotische und/oder antiinflammatorische Zusammensetzung gemäß einem der vorangehenden Ansprüche, wobei die Nanopartikel aus TiO₂ kristallin sind.

5. Antimikrobiotische und/oder antiinflammatorische Zusammensetzung gemäß einem der vorangehenden Ansprüche, wobei die festen Mikropartikel ausgewählt sind aus der Gruppe, bestehend aus Partikeln, die einen mittleren Partikeldurchmesser (D₅₀) von 120-180 µm haben.

6. Antimikrobiotische und/oder antiinflammatorische Zusammensetzung gemäß einem der vorangehenden Ansprüche, die die festen Mikropartikel bei einer Konzentration zwischen 0,5-20 g/l umfasst.

7. Antimikrobiotische und/oder antiinflammatorische Zusammensetzung gemäß einem der Ansprüche 1-6, wobei die festen Mikropartikel ausgewählt sind aus der Gruppe, bestehend aus TiO₂, Zirkoniumoxid, Diamantstaub, Polymeren, Polymilchsäure, Mineral, Keramik, Dialuminiumtrioxid, Calciumcarbonat, Calciumphosphat, Apatitkristallen, Knochenkeramikpartikeln, Titan, Zirkonium, Aluminiumoxid, Carborund, Bimsstein, Silica und Mischungen davon.

8. Antimikrobiotische und/oder antiinflammatorische Zusammensetzung gemäß einem der vorangehenden Ansprüche, die das H₂O₂ bei einer Konzentration von 3-7,5 Volumen-% umfasst.

9. Antimikrobiotische und/oder antiinflammatorische Zusammensetzung gemäß einem der vorangehenden Ansprüche, die weiterhin ein oder mehrere Detergens/Detergenzien, ausgewählt aus der Gruppe, bestehend aus SDS (Natriumdodecylsulfat) und SLS (Natriumlaurylsulfat), umfasst.

10. Antimikrobiotische und/oder antiinflammatorische Zusammensetzung gemäß einem der vorangehenden Ansprüche, wobei die Komponenten a), b) und c) vor der Auftragung und der letztendlichen Lagerung gemischt werden.

11. Antimikrobiotische und/oder antiinflammatorische Zusammensetzung gemäß einem der Ansprüche 1-9, wobei mindestens eine der Komponenten a), b) und c) getrennt gelagert wird und die Zusammensetzung direkt vor und/oder zum Zeitpunkt der Auftragung gemischt wird.

12. Kit, das einen ersten Behälter, der die Komponente a), wie in einem der Ansprüche 1-4 definiert, umfasst, einen zweiten Behälter, der die Komponente b), wie in einem der Ansprüche 1 und 8 definiert, umfasst, und einen dritten Behälter, der die Komponente c), wie in einem der Ansprüche 1 und 5-7 definiert, umfasst, optional zusammen mit Instruktionen für die Herstellung einer Zusammensetzung, wie in einem der Ansprüche 1-11 definiert, umfasst,
wobei das Kit optional weiterhin eine oder mehrere Vorrichtung (en) für die Auftragung der Zusammensetzung auf ein Individuum umfasst.

13. Kit gemäß Anspruch 12, wobei die Vorrichtung für die Auftragung der Zusammensetzung ein Implantatreinigungsund/oder Debridement-Werkzeug zum Reinigen und/oder Debridieren eines Implantats in der Mundhöhle ist,
wobei das Implantatreinigungs- und/oder Debridement-Werkzeug umfasst
a. ein verlängertes Basiselement, das aus mindestens zwei miteinander verdrillten Drähten gebildet ist, und
b. eine Vielzahl an Borsten, die zwischen den verdrillten Drähten befestigt sind und sich von den verdrillten Drähten weg erstrecken, wobei die Borsten in einem Reinigungsbereich an einem ersten Ende des Basiselements angeordnet sind; und wobei die Borsten aus Titan oder einer Titanlegierung bestehen.

14. Antimikrobiotische und/oder antiinflammatorische Zusammensetzung gemäß einem der Ansprüche 1-11 zur Verwendung als ein Medikament.

15. Antimikrobiotische und/oder antiinflammatorische Zusammensetzung gemäß einem der Ansprüche 1 bis 11 zur Verwendung für das Reinigen und/oder Debridieren eines Implantats in der Mundhöhle, einer harten Oberfläche in der Mundhöhle, einer Wunde in der Mundhöhle, eines parodontalen Defekts und/oder einer parodontalen Wunde und/oder eines oralen Hartgewebedefekts.

## Revendications

1. Composition antimicrobienne et/ou antiinflammatoire pour le nettoyage d'implant et/ou le débridement d'une surface dure dans la cavité buccale, qui comprend
a. des nanoparticules de TiO₂, ayant un diamètre moyen de particules (D₅₀) allant de 10 à 100 nm à une concentration comprise entre 0,5 et 500 g/L
b. H₂O₂ à une concentration d'au plus 7,5% en volume, et
c. des microparticules solides ayant un diamètre moyen de particules (D₅₀) allant de 100 à 200 µm à une concentration comprise entre environ 0,5 et 300 g/L.

2. Composition antimicrobienne et/ou antiinflammatoire selon la revendication 1, dans laquelle lesdites nanoparticules de TiO₂ sont choisies dans le groupe constitué de particules ayant un diamètre moyen de particules (D₅₀) allant de 20 à 30 nm.

3. Composition antimicrobienne et/ou anti-inflammatoire selon l'une quelconque des revendications précédentes, dans laquelle lesdites nanoparticules de TiO₂ sont présentes à une concentration allant d'environ 10 à 300 g/L

4. Composition antimicrobienne et/ou anti-inflammatoire selon l'une des revendications précédentes, dans laquelle lesdites nanoparticules de TiO₂ sont cristallines.

5. Composition antimicrobienne et/ou anti-inflammatoire selon l'une quelconque des revendications précédentes, dans laquelle lesdites microparticules solides sont choisies dans le groupe constitué de particules ayant un diamètre moyen de particules (D₅₀) allant de 120 à 180 µm.

6. Composition antimicrobienne et/ou anti-inflammatoire selon l'une des revendications précédentes, qui comprend lesdites microparticules solides à une concentration comprise entre 0,5 et 20 g/L.

7. Composition antimicrobienne et/ou anti-inflammatoire selon l'une quelconque des revendications 1 à 6, dans laquelle lesdites microparticules solides sont choisies dans le groupe constitué de TiO₂, d'oxyde de zirconium, de poussière de diamant, de polymères, d'acide polylactique, de minéral, de céramique, de trioxyde de dialuminium, de carbonate de calcium, de phosphate de calcium, de cristaux d'apatite, de particules céramiques osseuses, de titane, de zirconium, d'oxyde d'aluminium, de carborundum, de pierre ponce, de silice et de leurs mélanges.

8. Composition antimicrobienne et/ou anti-inflammatoire selon l'une quelconque des revendications précédentes, qui comprend ledit H₂O₂ à une concentration allant de 3 à 7,5% en volume.

9. Composition antimicrobienne et/ou anti-inflammatoire selon l'une quelconque des revendications précédentes, qui comprend en outre un ou plusieurs détergent(s) choisi(s) dans le groupe constitué de SDS (dodécylsulfate de sodium) et de SLS (laurylsulfate de sodium).

10. Composition antimicrobienne et/ou anti-inflammatoire selon l'une quelconque des revendications précédentes, dans laquelle lesdits composants a), b) et c) sont mélangés avant l'application et un stockage ultérieur.

11. Composition antimicrobienne et/ou anti-inflammatoire selon l'une quelconque des revendications 1 à 9, dans laquelle au moins l'un desdits composants a), b) et c) est stocké séparément et la composition est mélangée directement avant l'application et/ou au moment de l'application.

12. Kit comprenant un premier récipient comprenant un composant a) tel que défini dans l'une quelconque des revendications 1 à 4, un deuxième récipient comprenant un composant b) tel que défini dans l'une quelconque des revendications 1 et 8, et un troisième récipient comprenant un composant c) tel que défini dans l'une quelconque des revendications 1 et 5 à 7, facultativement, conjointement avec des instructions pour la préparation d'une composition telle que définie dans l'une quelconque des revendications 1 à 11,
ledit kit comprenant facultativement en outre un ou plusieurs dispositif(s) pour l'application de la composition à un sujet.

13. Kit selon la revendication 12, dans lequel le dispositif pour l'application de la composition est un outil de nettoyage et/ou de débridement d'implant pour nettoyer et/ou débrider un implant dans la cavité buccale,
ledit outil de nettoyage et/ou de débridement d'implant comprenant
a. un élément de base allongé formé d'au moins deux fils étant torsadés ensemble, et
b. une pluralité de poils fixés entre lesdits fils torsadés et s'étendant en s'éloignant desdits fils torsadés, moyennant quoi lesdits poils sont positionnés dans une section de nettoyage au niveau d'une première extrémité dudit élément de base ; et lesdits poils se composent de titane ou d'un alliage de titane.

14. Composition antimicrobienne et/ou anti-inflammatoire selon l'une quelconque des revendications 1 à 11 pour une utilisation en tant que médicament.

15. Composition antimicrobienne et/ou anti-inflammatoire selon l'une quelconque des revendications 1 à 11 pour une utilisation pour nettoyer et/ou débrider un implant dans la cavité buccale, une surface dure dans la cavité buccale, une plaie dans la cavité buccale, un défaut parodontal et/ou une plaie parodontale, et/ou un défaut de tissu dur buccal.
